# EUROPEAN PATENT APPLICATION

(11) **EP 4 403 737 A2**
(43) Date of publication of application: **24.07.2024**
(21) Application number: 23193705.3
(22) Date of filing: 28.08.2023
(51) Int. Cl.: E06B 3/263, E06B 3/36, E06B 3/82, E06B 3/88, E06B 7/23, E06B 3/70, E05D 7/081

(54) **THERMALLY INSULATED DOOR ASSEMBLY**

(30) Priority: 07.10.2022 KR 20220128707; 22.06.2023 KR 20230080503
(71) Applicant: Min, Ju Hong, Seo-gu Daejeon (KR)
(72) Inventor: Min, Ju Hong, Seo-gu Daejeon (KR)
(74) Representative: M. Zardi & Co S.A.

(57) **Abstract**

The present invention relates to a thermally insulated door assembly capable of improving thermal insulation performance by improving sealing between a door leaf and a door frame, and more particularly, to a thermally insulated door assembly capable of increasing the thermal insulation effect by blocking external cold wind and noise flowing in between a door frame and a door leaf.

## Description

### BACKGROUND

### 1. Field

The present invention relates to a thermally insulated door assembly capable of improving thermal insulation performance by improving sealing between a door leaf and a door frame, and more particularly, to a thermally insulated door assembly capable of increasing the thermal insulation effect by blocking external cold wind and noise flowing in between a door frame and a door leaf.

### 2. Description of Related Art

In general, a front entrance refers to an entrance area of a building. Therefore, the front entrance serves as a point of connection between the interior and exterior of the building, and a method of manufacturing a door frame and a door of the front entrance using aluminum profiles is recently widely used.

An entrance door installed at the front entrance may include a door frame, a door rotatably coupled to the doorframe, and the door may have a structure that is coupled to a hinge formed on the doorframe and rotates. Therefore, as the door rotates, the open part of the front entrance may be open and closed. The entrance door installed in the front entrance is exposed to the outside and serves as the face of the building. As the entrance may significantly impact the impression of the building, an entrance door installed in the entrance is often required to be decorated to be aesthetically appealing.

In a conventional entrance door, due to the nature of a front entrance, the inner side of the door is connected to the building's interior, while the outer side of the door is connected to the exterior of the building, so when a temperature difference occurs between the building's interior and exterior due to heating and cooling facilities, outside air and inside air are replaced between the door and the doorframe, which may diminish the heating and cooling efficiency. In other words, inadequate sealing between the door and the doorframe may create a gap through which air flows, reducing the heating and cooling efficiency.

Therefore, it is necessary to develop a door that seals the space between the door and a doorframe, particularly, between the door and the doorframe where a hinge is formed, to prevent outside air from entering when the door is closed.

### [Related Art Documents]

### [Patent Documents]

(Patent Document 1) KR 10-1067759 B1
(Patent Document 2) KR 10-1433569 B1
(Patent Document 3) KR 10-1828148 B1
(Patent Document 4) KR 10-2034978 B1
(Patent Document 4) KR 10-2034978 B1

### SUMMARY

This summary is provided to introduce a selection of concepts in a simplified form that are further described below in the Detailed Description. This summary is not intended to identify key features or essential features of the claimed subject matter, nor is it intended to be used as an aid in determining the scope of the claimed subject matter.

The present invention has been made to solve the above problems, and an object of the present invention is to provide a thermally insulated door assembly capable of preventing outside air from entering into an indoor space by sealing a space between a door and a door frame.

In one general aspect, a thermally insulated door assembly may include a doorframe 100 installed in an opening and including hinge shafts 110 installed respectively on upper and lower sides thereof; a door 200 configured to open and close the opening; hinge covers 300 formed respectively on upper and lower sides of the door 200 and having bearings 310 into each of which the hinge shaft 110 is inserted; and a sealing cover 400 formed on a side surface of the door 200 adjacent to the hinge shafts 110.

In addition, each of the hinge covers 300 may include a first metallic hinge cover frame 341, a second metallic hinge cover frame 342, and a hinge cover insulation portion 340 configured to connect the first and second hinge cover frames 341 and 342.

In addition, the door 200 may further include a door frame 220 which surrounds the door body 210, is fixed to the door body 210, and has a first insulation portion 221 formed therein.

In addition, the first insulation portion 221 may include a first insulation plate 221-1 configured to partition an inner space of the first insulation portion 221; and a second insulation plate 221-2 spaced apart by a predetermined distance from the first insulation plate 221-1.

In addition, the door 200 may include a frame cover 230 spaced apart by a predetermined distance from the door frame 220; and a pair of finishing plates 240 fixed to fixing protrusions 220-1 and 230-1 respectively formed on the door frame 220 and the frame cover 230.

In addition, a second insulation portion 231 disposed outside the door frame 220 may be further included.

In addition, the second insulation portion 231 may include an inner frame 231-1 provided between the outside of the door frame 220 and the frame cover 230; side insulation portions 231-2 formed on both sides of the inner frame 231-1 to seal a space between the door frame 220 and the frame cover 230 and having an air layer a formed therein; and close-contact insulation portions 231-3, respectively, in close contact with a front surface and a rear surface of the inner frame 231-1 to reduce conductivity of heat to the frame cover 230 and the door frame 220.

Also, the bearing 310 may be seated on a bearing bar 350.

In addition, the hinge cover 300 may be disposed between the bearing bar 350 and the inner frame 231-1 and fixed to the bearing bar 350 and the inner frame 231-1.

In addition, the sealing cover 400 may include a first cover portion 140 configured to surround a door body 210 and having one side fixed to a door frame 220 fixed to the door body 210; and a second cover portion 420 spaced apart by a predetermined distance from the door frame 220 and having one side fixed to the door frame 220 or a frame cover 230.

In addition, the sealing cover 400 may further include a connection cover portion 430 configured to connect the first and second cover portions.

In addition, the thermally insulated door assembly may further include an outside air blocking portion 500 installed between the doorframe 100 and the sealing cover 400 to block outside air.

In addition, the outside air blocking portion 500 may include a fixed body 510 fixed to a side surface of the doorframe 100; and a rotating body 520 fixed to the first cover portion 410 and configured to come in contact with or apart from the fixed body 510 according to the opening and closing of the door 200.

In addition, a plurality of recessed portions and groove portions may be formed on each of contact surfaces of the fixed body 510 and the rotating body 520.

Also, the second cover portion 420 may be rotatably coupled to the frame cover 230.

In addition, the thermally insulated door assembly may further include an elastic support portion 440 configured to elastically support the second cover portion 420 between the first cover portion 410 and the door frame 220.

In addition, the elastic support portion 440 may include a first elastic body 441 and a second elastic body 442 to support one side of the first cover portion 410 uniformly from both sides.

In addition, a first gasket 550 may be provided on an inner surface of the doorframe 100, a second gasket 560 may be provided on a side surface of the door 200, the second gasket 560 may be disposed adjacent to the sealing cover 400, the first and second gaskets 550 and 560 may be formed of an insulating material having elasticity, and the first and second gaskets 550 and 560 may be in contact with each other when the door 200 is closed.

In addition, one side of the first gasket 550 may be formed in a shape that surrounds an exposed portion of the doorframe 100 facing the side of the door 200, the first and second gaskets 550 and 560 may have a hollow interior, the first and second gaskets 550 and 560 may have partition walls crossing the hollow interior, the first and second gaskets 550 and 56 may have curvatures corresponding to each other and may be in contact with each other, the first gasket 550 may have a protrusion portion protruding from the other side thereof, the doorframe 100 may include an auxiliary gasket 570 in contact with a corner portion of the door 200 and disposed adjacent to the protrusion portion of the first gasket 550.

In addition, the thermally insulated door assembly may further include an outside air blocking portion 500-1 installed between the doorframe 100 and the sealing cover 400 to block outside air.

In addition, the outside air blocking portion 500-1 may include a fixed blocking film 530 installed inside the doorframe 100 and having an end in close contact with an outer side surface of the sealing cover 400; and a rotation blocking film 540 installed on the outer side surface of the sealing cover 400 and having an end in contact with an inner side surface of the doorframe 100.

In addition, the fixed blocking film 530 may be provided in plurality, and the rotation blocking film 540 may be provided in plurality.

In addition, the plurality of rotation blocking films 540 may be positioned between at least some of the plurality of fixed blocking films 530.

In another general aspect, a thermally insulated door assembly may include a doorframe 100 installed in an opening; a door 200 configured to open and close the opening; a door frame 220 disposed on an outward side of the door 200 and firmly fixed to a door body 210; a pair of fixing bars 223 symmetrical to each other and installed inside the door frame 220; a first insulation portion 221 disposed between the pair of fixing bars 223 and inserted into and fixed to an end of each fixing bar 223 of the pair; and a plate-shaped frame cover 230 including a third insulation portion 234 on an indoor side of the door 200 and spaced apart by a predetermined distance from the door frame 220.

In addition, the thermally insulated door assembly may include a first side insulation portion 235 and a second side insulation portion 236 that are disposed on both sides of the frame cover 230 and configured to seal a space between the frame cover 230 and the door frame 220; a pair of fixing protrusions 220-1 and 230-1 respectively formed on the door frame 220 and the frame cover 230 such that one fixing protrusion of the pair is disposed on the outer side of the door 200 and the other fixing protrusion is on the indoor side of the door 200; and a first gasket 550 on an inner side surface of the doorframe 100, and a second gasket 560 may be provided on a side surface of the door 200.

In addition, the second side insulation portion 236 and the second gasket 560 that are adjacent to the doorframe 100 may be configured to form a single body together.

In addition, the first gasket 550 may be configured to form a single body with a fixing gasket 140 installed inside the doorframe 100.

In still another general aspect, a thermally insulated door assembly may include a doorframe 100 installed in an opening and including hinge shafts 110 installed respectively on upper and lower sides thereof; a door 200 configured to open and close the opening; and a sealing cover 400 formed on a side surface of the door 200 adjacent to the hinge shafts 110, wherein the sealing cover 400 includes a first cover portion 140 configured to surround a door body 210 and having one side fixed to a door frame 220 fixed to the door body 210; and a second cover portion 420 spaced apart by a predetermined distance from the door frame 220 and having one side fixed to the door frame 220 or a frame cover 230.

In yet another general aspect, a thermally insulated door assembly may include a doorframe 100 installed in an opening and including hinge shafts 110 installed respectively on upper and lower sides thereof; a door 200 configured to open and close the opening; and hinge covers 300 formed respectively on upper and lower sides of the door 200 and having bearings 310 into each of which the hinge shaft 110 is inserted, wherein each of the hinge covers 300 includes a first metallic hinge cover frame 341, a second metallic hinge cover frame 342, and a hinge cover insulation portion 340 configured to connect the first and second hinge cover frames 341 and 342.

In still another general aspect, a thermally insulated door assembly may include a doorframe 100 installed in an opening; and a door 200 configured to open and close the opening, wherein the door 200 includes a door frame 220 surrounding a door body 210, fixed to the door body 210, and having a first insulation portion 221 formed therein and the first insulation portion 221 includes a first insulation plate 221-1 configured to partition an inner space of the first insulation portion 221; and a second insulation plate 221-2 spaced apart by a predetermined distance from the first insulation plate 221-1.

Other features and aspects will be apparent from the following detailed description, the drawings, and the claims.

### BRIEF DESCRIPTION OF THE DRAWINGS

FIGS. 1 and 2 are cross-sectional views showing the overall structure of a thermally insulated door assembly according to an embodiment of the present invention;
FIG. 3 is a cross-sectional view showing a main configuration of a door of FIG. 1;
FIG. 4 is a cross-sectional view showing one embodiment of opening and closing of the door of FIG. 1 and one embodiment of an operation of an outside air shut-off portion of the door of FIG. 1;
FIG. 5 is a partial cross-sectional view showing another embodiment of a sealing cover of FIG. 1;
FIGS. 6 and 7 are cross-sectional views showing a partial structure of a thermally insulated door assembly according to another embodiment of the present invention;
FIG. 8 is a cross-sectional view showing another embodiment of a door of the present invention; and
FIGS. 9 and 10 are, respectively, a cross-sectional view and a partial cross-sectional view showing one embodiment of first and second gaskets of the present invention.

Throughout the drawings and the detailed description, unless otherwise described, the same drawing reference numerals will be understood to refer to the same elements, features, and structures. The relative size and depiction of these elements may be exaggerated for clarity, illustration, and convenience.

### DETAILED DESCRIPTION

Hereinafter, embodiments of the present disclosure will be described in detail with reference to the drawings.

In the specification, terms such as "first" and "second" are used herein to arbitrarily distinguish between the elements such terms describe and are not intended to indicate or imply relative importance or significance. Such terms should be used only for the purpose of distinguishing one element from another. For example, the 'first' component may be named the 'second' component without departing from the scope of the present invention, and the 'second' component may also be similarly named the "first" component. The term "and/or" includes a combination of a plurality of items or any one of a plurality of terms.

It will be understood that when an element is "connected to" or "coupled with/to" to another element, the element may be directly connected or coupled to another element, and there may be an intervening element between the element and another element. On the other hand, it will be understood that when an element is "directly connected" or "directly coupled" to another element, there is no intervening element between the element and another element. In addition, it is understood that when a first element is connected to or accesses a second element in a network, the first element and the second element can transmit and receive data therebetween.

In addition, suffixes "module" and "unit" for elements as used herein are given in consideration of ease in preparing the present specification only, and do not have a particularly important meaning or role in themselves. Accordingly, the terms "module" and "unit" may be used interchangeably.

When such elements are implemented in an actual application, if necessary, two or more elements may be combined into one element, or one element may be subdivided into two or more elements. Throughout the drawings, identical or similar elements are denoted by the same reference numeral, and detailed descriptions of elements having the same reference numeral may be omitted and replaced with the description of the previously described element.

Moreover, the present invention covers all possible combinations of example embodiments indicated in this specification. It is to be understood that the various embodiments of the present invention, although different, are not necessarily mutually exclusive. A particular feature, structure, or characteristic described herein in connection with one embodiment may be implemented within other embodiments without departing from the spirit and scope of the present disclosure. For example, elements mentioned in first and second embodiment may perform all functions described in the first and second embodiments.

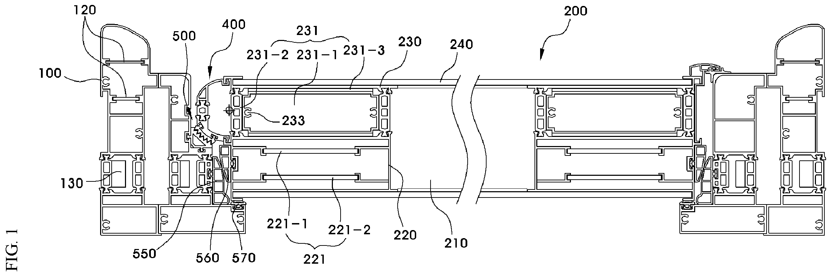

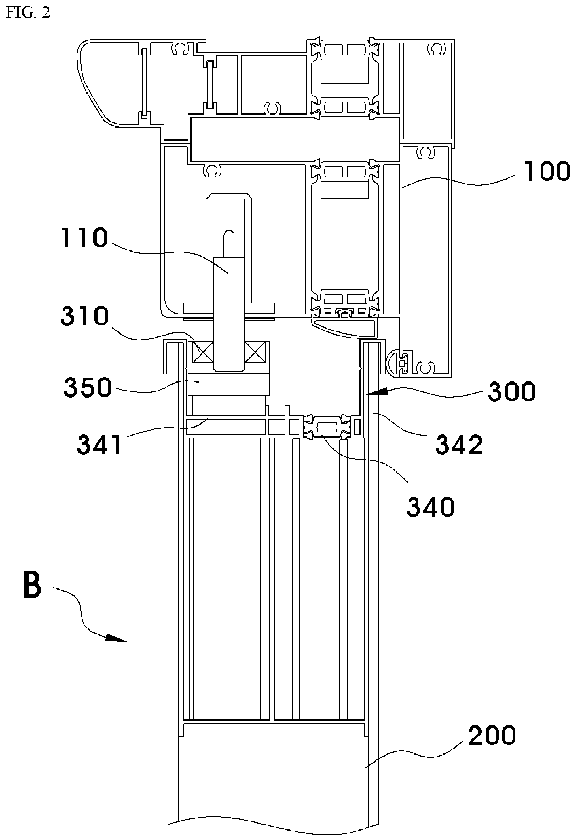

Referring to FIGS. 1 and 2, the present invention may include a doorframe 100, a door 200, hinge covers 300, a sealing cover 400, and an outside air blocking portion 500.

The doorframe 100 is installed in the form of a rectangular frame along the edge of an opening formed in a building. Hinge shafts 110 may be installed on upper and lower sides of the doorframe 100, respectively. The door shaft 110 corresponds to a rotation shaft around which the door 200 rotates. The hinge shafts 110 may be firmly fixed to the inside of the doorframe 100.

Inside the doorframe 100, a first internal insulation member 120 and a second internal insulation member 130 may be provided along the shape of the doorframe 100. The doorframe 100 may have a profile-type shape manufactured by extrusion, wherein the inside of the doorframe 100 is hollow, which is vulnerable to thermal insulation. The first and second internal insulation members 120 and 130 may solve the issues with thermal insulation.

The first internal insulation member 120 may be formed on one side of the doorframe 100 exposed to the outside and made of a polyamide material. The first internal insulation member 120 may be provided in plurality to be spaced apart by a predetermined distance. In addition, the second internal insulation member 130 may be a phenolic foam (closed cell) with high thermal insulation and semi-non-combustible performances obtained by foaming a thermosetting resin capable of filling the hollow inside of the doorframe 100 at a closed cell rate. This may further improve the thermal insulation performance.

The door 200 is rotatably installed on the doorframe 100 to open and close the opening. The hinge shafts 110 are coupled to the upper and lower sides of the door 200, allowing the door 200 to freely rotate and open and close the opening.

The hinge covers 300 may be respectively disposed on each of the upper and lower sides of the door 200. The hinge cover 300 may have a bottom surface firmly fixed to the upper, lower, and side surfaces of the door 200 while forming a "U" shape. Inside the hinge cover 300 recessed in the U-shape, a bearing 310 may be integrally fixed to the hinge cover 300. The hinge shaft 110 is inserted into the bearing 310. When the bearing 310 is inserted into the hinge shaft 110, the door 200 may be stably fixed to the doorframe, and as the hinge shaft 110 is inserted into the bearing 310, the door 200 may be rotated. Here, the bearing 310 may be a ball bearing, or the like.

A bearing bar 350 on which the bearing 310 is seated may be firmly fixed into an inner frame threaded hole 233 of an inner frame 231-1 with a screw or a bolt. The bearing bar 350 may be firmly fixed to the hinge cover 300.

The hinge cover 300 may be disposed between the bearing bar 350 and the inner frame 231-1. The hinge cover 300 may be fixedly coupled to the bearing bar 350 and the inner frame 232-1.

The hinge cover 300 may be provided with first and second metallic hinge cover frames 341 and 342 and a hinge cover insulation portion 340 configured to connect the first and second hinge cover frames 341 and 342. The hinge cover insulation portion 340 may block heat transferred to the first and second metallic hinge cover frames 341 and 342.

The hinge cover 300 may be provided with first and second metallic hinge cover frames 341 and 342 and a hinge cover insulation portion 340 configured to connect the first and second hinge cover frames 341 and 342. The hinge cover insulation portion 340 may block heat transferred to the first and second metallic hinge cover frames 341 and 342.

The sealing cover 400 may be installed on a side surface of the door 200 adjacent to the hinge shaft 110. The sealing cover 400 may partially block a space between the side surface of the door 200 and the doorframe 100 when the door 200 is opened. The sealing cover 400 may extend to a length equal to the vertical length of the door 200.

The sealing cover 400 rotates integrally with the door 200, and the sealing cover 400 preferably has a structure that creates a predetermined radius of curvature so that it does not interfere with the doorframe 100 during the rotation.

The outside air blocking portion 500 may be installed on the sealing cover 400. The outside air blocking portion 500 may be installed between the sealing cover 400 and the doorframe 100 in order to block outside air entering into the space between the sealing cover 400 and the doorframe 100. When the door 200 is open, outside air passes through the outside air blocking portion 500, and when the door 200 is closed, the outside air blocking portion 500 may block the outside air entering into the gap between the door 200 and the doorframe 100.

Referring to FIG. 1, the door 200 may include a door body 210, a door frame 220, a frame cover 230, and finishing plates 240.

The door body 210 may have a structure filled with an insulating material therein, and may be formed in the shape of a rectangular plate. The insulating material filling the inside of the door body 210 may be preferably a non-combustible material.

The door frame 220 may form a rectangular shape encircling the edges of the door body 210, and may be firmly fixed to the door body 210. The door frame 220 may be a frame for providing a shape of the door 200 and a predetermined rigidity to the door body 210, and may be produced in the form of an extruded profile. Accordingly, the interior of the door frame 220 forms a hollow shape, and a first insulation portion 221 may be disposed inside the door frame 220.

The frame cover 230 may be spaced apart from the door frame 220 by a predetermined distance so as to further include a second insulation portion 231 outside the door frame 220. That is, the distance by which the frame cover 230 is spaced apart may vary depending on the thickness of the door body 210. When the frame cover 230 includes the second insulation portion 231, the thermal insulation performance of the door frame 220 may be further improved.

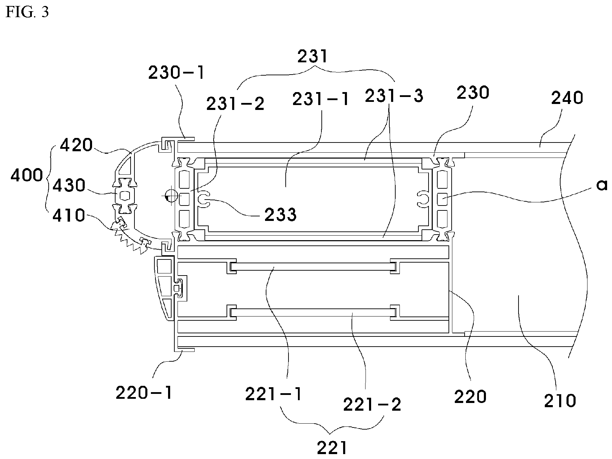

Referring to FIG. 3, the finishing plates 240 may be fixed to fixing protrusions 220-1 and 230-1 formed respectively on the door frame 220 and the frame cover 230. The finishing plates 240 may have a plate shape with a predetermined thickness and may be made of a material with a predetermined strength for the purpose of preventing damage to the door body 210. It is preferable that a pair of finishing plates 240 provided on both sides of the door body 210.

Referring to FIGS. 1 and 3, the first insulation portion 221 may include a first insulation plate 221-1 and a second insulation plate 221-2. The first insulation plate 221-1 may be formed to have the same length as the extension length of the door frame 220 so as to partition the hollow interior of the first insulation portion 221, i.e., the door frame 220. The second insulation plate 221-1 may be installed at a position apart by a predetermined distance from the first insulation plate 221-1 and may be made of a polyamide material, further improving the thermal insulation performance.

Referring to FIGS. 1 and 3, the second insulation portion 231 may include an inner frame 231-1, side insulation portions 231-2, and close-contact insulation portions 231-3.

The inner frame 231-1 may be provided between the outer side of the door frame 220 and the frame cover 230 and installed for the purpose of providing higher rigidity to the door 200. In other words, when perforation is performed to install a handle or a door lock on the edge of the door 200, the inner frame 231-1 may prevent a decrease in the rigidity of the door 200 due to the perforation.

In addition, a predetermined space between the frame cover 230 and the door frame 220 may be provided through the inner frame 231-1. The inner frame 231-1 may also be produced in the form of an extruded profile and may extend along the door frame 220 to a predetermined length.

The inner frame 231-1 may be coupled to the frame cover 230 on an outer side of the door with a screw or the like. A screw head on the outer side B of the door may not be visible from the outside by the finishing plate 240.

The side insulation portions 231-2 are formed respectively on both sides of the inner frame 231-1 and seal the space between the door frame 220 and the frame cover 230. The side insulation portions 231-2 may be formed of a polyamide material and may seal the space between the door frame 220 and the frame cover 230 to provide a predetermined thermal insulation effect. The side insulation portions 231-2 may form an air layer a therein to further improve the thermal insulation performance.

In particular, when perforation is performed on the side surface of the door 200 to install a handle, hinge, or door lock of the door 200 and outside air enters through the gap thereof, the sealed space between the door frame 220 and the frame cover 230 may prevent the outside air from entering the indoor space and increases the length of the path along which the outside air flows in, further improving the thermal insulation performance.

The close-contact insulation portions 231-3 may be provided respectively on the front and rear surfaces of the inner frame 231-1. That is, the close-contact insulation portions 231-3 may be respectively provided between the front surface of the inner frame 231-1 and the frame cover 230 and between the rear surface of the inner frame 231-1 and the door frame 220. Referring to the drawings, each member may be in close contact with both sides of the close-contact insulation portion 231-3 and the close-contact insulation portions 231-3 may be made of a material having a thermal insulation effect, such as polyamide. Through this, the conductivity of heat to the door frame 220 through the frame cover 230 may be reduced, thereby improving the thermal insulation performance.

Referring to FIGS. 1 and 3, the sealing cover 400 may include a first cover portion 410, a second cover portion 420, and a connection cover portion 430.

The first cover portion 410 may be fixed on one side of the door frame 220 and fixed in a structure that is detachable from one side of the door frame 220. The first cover portion 410 may be formed to create a predetermined radius of curvature.

The second cover portion 420 may be fixed on one side of the frame cover 230 and fixed in a detachable structure. The second cover portion 420 is also preferably formed to create a predetermined radius of curvature.

The connection cover portion 430 may is purposed to connect the first and second covers 410 and 420 to each other and may be preferably made of a material having a predetermined elasticity (rubber, urethane, silicone, etc.). That is, the facing ends of the first cover portion 410 and the second cover portion 420 are spaced apart by a predetermined distance, and the connection cover portion 430 may be disposed in the space therebetween and may connect the first and second covers 410 and 420 to each other.

In addition, as the connection cover part 430 is formed of a material having elasticity, it may serve as a predetermined cushion when a hand or a foreign substance is trapped between the sealing cover 400 and the doorframe, thereby preventing damage to the door 200 or the doorframe 100.

That is, the side surface of the door 200 is prevented from being exposed to the outside through the sealing cover 400, and in particular, when the door 200 is opened, the space between the door 200 and the doorframe is blocked to reduce the risk of safety accidents, such as foreign substances or a hand being trapped in the gap.

The inner side of the doorframe 100 and the side surface of the door 200 adjacent to the doorframe 100 are in close contact with each other when the door 200 is closed, but are not completely sealed due to the gap of a predetermined distance, which may allow outside air to flow in. To prevent this, the doorframe 100 may be provided with first gaskets 550 disposed on the inner side surfaces and the door 200 may be provided with second gaskets 560 disposed on the side surfaces of the door 200 adjacent to the doorframe 100. The first and second gaskets 550 and 560 may be respectively disposed on both side surfaces of the door 200 and on both inner side surfaces of the doorframe 100 adjacent to the door 200. The first and second gaskets 550 and 560 is preferably formed of an insulating material and preferably has elasticity.

When the door 200 is closed, the first gasket 550 and the second gasket 560 come into contact with each other to seal the gap therebetween and block outside air from flowing in. To block outside air, the second gasket 560 is preferably disposed adjacent to the sealing cover 400. The first and second gaskets 550 and 560 may be disposed to be atleast partially in contact with each other.

The first gasket 550 may be formed in a shape in which one side thereof surrounds the exposed portion of the doorframe 100 facing the side surface of the door 200. In other words, the doorframe 100 is made of a metallic material and the first gasket 550 is formed to surround the exposed portion of the doorframe 100, preventing heat loss due to heat transfer applied to the metallic material.

One sides of the first and second gaskets 550 and 560 may have curvatures corresponding to each other and may contact each other. When the first and second gaskets 550 and 560 come into contact with each other, they become flatter than their original shapes, thereby further improving the sealing effect. The first gasket 550 may include a protrusion portion protruding from the other side thereof. The protrusion portion of the first gasket 550 may come into contact with the exposed portion of the doorframe 100 to further help block outside air.

The first gasket 550 and the second gasket 560 may form a shape having a hollow interior such that their shapes can deform when in contact with each other. In addition, the first and second gaskets 550 and 560 may be provided with partition walls crossing their hollow interiors to maintain a predetermined rigidity. The partition walls allow the rigidity and elasticity of the first and second gaskets 550 and 560 to be maintained, so that the adhesion between the first and second gaskets 550 and 560 can be increased and a phenomenon such as the entry of outside air can be prevented even when used for long periods.

In addition, the doorframe 100 may be provided with an auxiliary gasket 570 capable of blocking outside air from the corner portions of the door 200 by contacting the corner portions when the door 200 is closed. With the auxiliary gasket 570, the adhesion between the doorframe 100 and the door 200 may be further improved. The auxiliary gasket 570 may be disposed to be adjacent to or in contact with the protrusion portion of the first gasket 550.

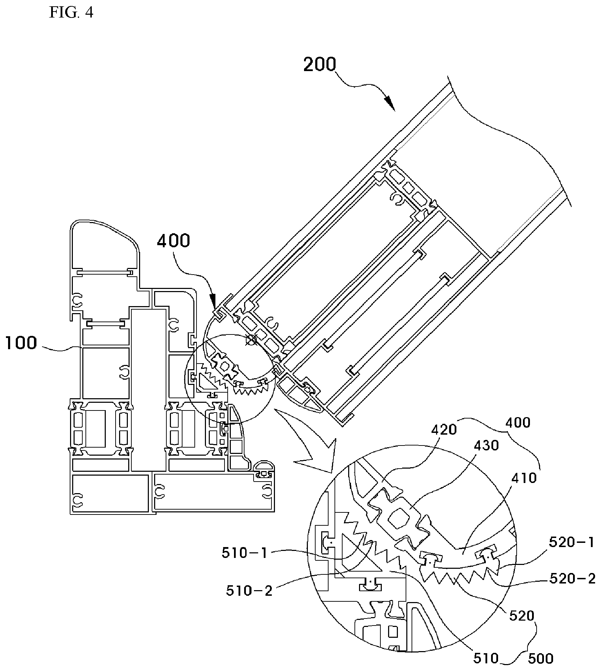

Referring to FIG. 4, the outside air blocking portion 500 may include a fixed body 510 and a rotating body 520. The fixed body 510 may be fixed to the side surface of the doorframe 100 and formed of a material with elasticity. The rotating body 520 may be fixed to the first cover portion 410 and rotates along with the door 200 such that the rotating body 520 may come in contact with or apart from the fixed body 510 according to the opening and closing of the door 200.

That is, when the door 200 is opened, the rotating body 520 rotates and is spaced apart from the fixed body 510 by a predetermined distance, and when the door 200 is closed, the rotating body 520 gradually comes into contact with the fixed body 510, thereby preventing outside air from entering through the gap between the door 200 and the doorframe 100.

In this case, a plurality of recessed portions 510-1 and 520-1 and a plurality of groove portions 510-2 and 520-2 may be formed on each contact surface between the rotating body 520 and the fixed body 510. The recessed portions 510-1 and 520-1 and the groove portions 510-2 and 520-2, which are respectively formed on the fixed body 510 and the rotating body 520 are engaged with each other to increase the adhesion between the fixed body 510 and the rotating body 520, thereby effectively blocking outside air.

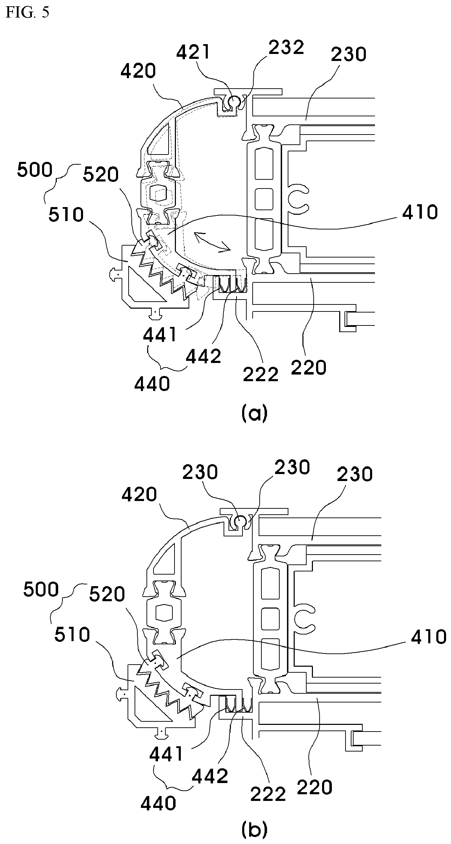

An embodiment of the present invention will be described in detail with reference to FIG. 5.

The second cover portion 420 may be rotatably coupled to the frame cover 230. In this case, a rotary shaft 421 may be formed on the second cover portion 420, the rotary shaft 421 may be inserted into the frame cover 230, and a rotary shaft bracket 232 surrounding the rotary shaft 421 may be formed on the frame cover 230. With this structure, the second cover portion 420 may be rotatably coupled to the frame cover 230.

In addition, an elastic support portion 440 configured to elastically support the first cover portion 410, the second cover portion 420, and the connection cover portion 430 may be further formed between the first cover portion 410 and the door frame 220 to allow the second cover portion 420 to rotate within a predetermined range of angles. The elastic support portion 440 may be installed on a bracket 222 provided on ones side of the first cover portion 410. Due to this structure, when the sealing cover 400 is pressed with a predetermined pressure, the sealing cover 400 may rotate around the rotary shaft 421, and upon removal of the pressing force, the sealing cover 400 may be restored to its original position by means of the elastic support portion 440.

In more detail, a predetermined clearance or space may be provided to allow the sealing cover 400 to rotate around the rotary shaft 421. With the clearance or space allowing for the rotation of the sealing cover 400, the recessed portions 510-1 and 520-1 and the groove portions 510-2 and 520-2 may be smoothly engaged and slide when the door 200 is opened and closed.

That is, a predetermined clearance or space is required in order for one of the plurality of recessed portions 510-1 and 520-1 to slide into another adjacent groove 510-2 or 520-2, and the sealing cover 400 may provide the necessary clearance or space while rotating, so that the recessed portions 510-1 and 520-1 and the groove portions 510-1 and 520-2 can be smoothly engaged with each other.

Also, the elastic support portion 440 may include a first elastic body 441 and a second elastic body 442. The first and second elastic bodies 441 and 442 may elastically support one side of the first cover portion 410 uniformly from both sides. Accordingly, the sealing cover 400 may be stably supported so that occurrences such as dislodgement of the sealing cover 400 due to repeated movements can be prevented.

According to the present invention with this configuration, the space between the door 200 and the doorframe 100 may be effectively blocked, thereby improving thermal insulation performance and thus reducing cooling and heating costs.

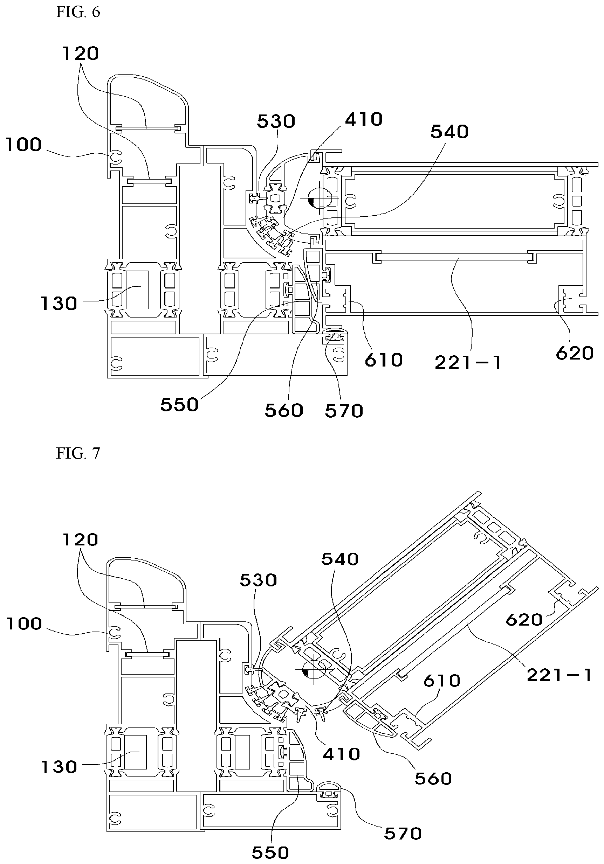

FIGS. 6 and 7 are cross-sectional views showing a partial structure of a thermally insulated door assembly according to another embodiment of the present invention. The following description will be given with reference to FIGS. 1 to 5. FIGS. 6 and 7 illustrate another embodiment of blocking of outside air between the doorframe and the sealing cover, unlike the embodiment of FIGS. 1 to 5. Descriptions of elements having the same reference numerals will be omitted.

Referring to FIGS. 6 and 7, in another embodiment, the outside air blocking portion 500-1 may include a fixed blocking film 530 formed of a material with elasticity and a rotation blocking film 540 to block outside air between the doorframe 100 and the sealing cover 400.

The fixed blocking film 530 may be installed inside the doorframe 100 adjacent to the outdoor space and may extend along the longitudinal direction of the doorframe 100. An end of the fixed blocking film 530 may be in close contact with the outer side surface of the sealing cover 400. The fixed blocking film 530 may be provided in plurality to be spaced apart by a predetermined distance.

The rotation blocking film 540 may be installed on the outer side surface of the sealing cover 400. An end of the sealing cover 400 may be in contact with the inner side surface of the doorframe 100. The rotation blocking film 540 may be provided in plurality. The rotation blocking film 540 may be positioned between at least some of the plurality of fixed blocking films 530.

Referring to FIG. 6, when the door is closed, the fixed blocking films 530 and the rotation blocking films 540 are arranged in an overlaying fashion in the space between the sealing cover 400 and the doorframe, so that the entry of outside air can be blocked.

Referring to FIG. 7, when the door is opened, the fixed blocking film 530 remains in contact with the outer side surface of the sealing cover 400, blocking outside air. To this end, the fixed blocking film 530 is preferably arranged mainly on the inner side, that is, around the first cover portion 410. Accordingly, the rotation blocking film 540 is preferably installed on the first cover portion 410.

The doorframe 100 and the door 200 may be provided with at least three insulation portions for blocking heat transfer. The insulation plates 221-1 and 221-2, the side insulation portions 231-1 and 231-2, and the close-contact insulation portion 231-3, which are described above, may belong thereto. In addition, referring to FIGS. 6 and 7, the insulation portions for blocking heat transfer may be further provided with an adhesive fixed insulation form 610.

The adhesive fixed heat insulation form 610 may be a part constituting the door frame 220 or the doorframe 100, and may have a foam filling space 620 therein.

In the door frame 220, the adhesive fixed insulation form 610 may divide one section of the door frame 220 into an upper part and a lower part. Two or more adhesive fixed insulation forms 610 are preferably disposed in one section of the door frame 220.

The adhesive fixed insulation form 610 may be filled with liquid foam such as Azone, and when the liquid foam is hardened, a part thereof may be removed to provide an insulation function.

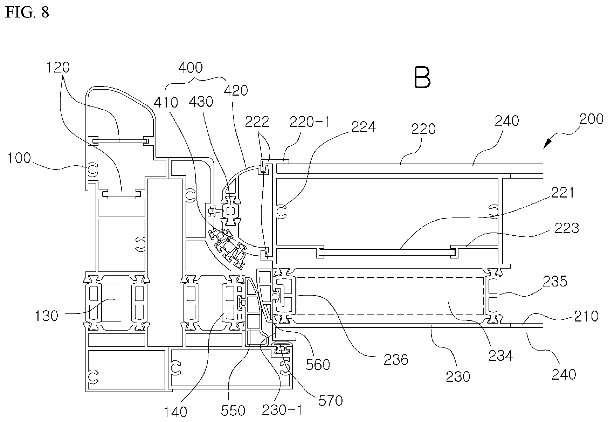

Another embodiment of the door 200 of the present invention will be described with reference to FIG. 8.

The door 200 may include a door body 210, a door frame 220, a frame cover 230, and finishing plates 240.

The door body 210 is the same as the door body 210 described in detail above.

The door frame 220 is installed on the outward side of the door. The door frame 220 forms a rectangular shape enclosing the edges and has a shape having a hollow interior. The door frame 220 may be firmly fixed to the door body 210. The door frame 220 may be a frame for providing a shape of the door 200 and a predetermined rigidity to the door body 210, and may be produced in the form of an extruded profile.

Meanwhile, a pair of fixing bars 223 symmetrical to each other are further formed inside the door frame 220. The first insulation portion 221 is disposed between the pair of fixing bars 223 and the first insulation portion 221 is fixed by being inserted into each end of the pair of fixing bars 223.

In addition, a threaded hole 224 may be further formed on the inner side surface of the door frame 220. A separate screw or bolt may be inserted into the threaded hole 224 to fix a bearing bar 350 on which the bearing 310 is seated.

A pair of brackets 222 symmetrical to each other and spaced apart by a predetermined distance from each other are further formed on one side of the door frame 220. One sides of the first cover portion 410 and the second cover portion 420 of the sealing cover 400 are respectively inserted into the brackets 222.

The frame cover 230 is spaced apart by a predetermined distance from the door frame 220 to further include a third insulation portion 234 on the indoor side of the door 200. The frame cover 230 is in the form of a plate having a predetermined thickness. A first side insulation portion 235 and a second side insulation portion 236 for sealing a space between the frame cover 230 and the door frame 220 are further formed on both sides of the frame cover 230.

A pair of finishing plates 240 are provided and fixed to fixing protrusions 220-1 and 230-1 respectively formed on the door frame 220 and the frame cover 230 such that one finishing plate 240 is disposed on the outside of the door 200 and the other finishing plate 240 is disposed on the indoor side of the door 200.

On the other hand, the second side insulation portion 236 and the second gasket 560 adjacent to the doorframe 100 may have structures that can be coupled to each other, or may form a single body together.

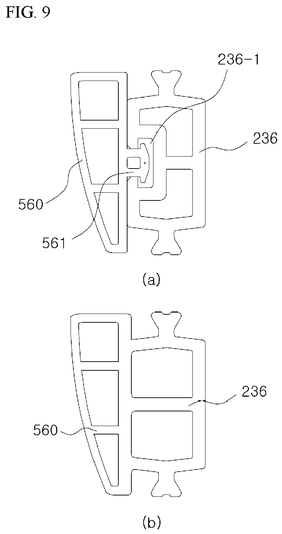

Referring to (a) of FIG. 9, as an example of a structure in which the second side insulation portion 236 and the second gasket 560 can be coupled to each other, a coupling protrusion 561 is further formed on one side of the second gasket 560 and a fastening hole 236-1 into which the coupling protrusion 561 can be inserted is further formed in the second side insulation portion 236.
(b) of FIG. 9 illustrates an example in which the second side insulation portion 236 and the second gasket 560 form a single body together.

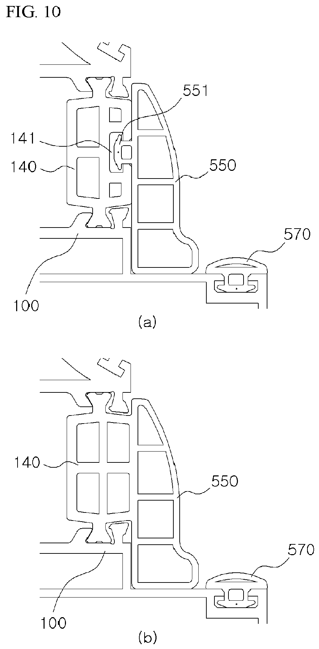

Referring to FIG. 10, the first gasket 550 may be coupled to a fixing gasket 140 installed inside the doorframe 100 or may be formed to form a single body with the fixing gasket 140.

As an embodiment, referring to (a) of FIG. 10, a coupling protrusion protrudes from one side of the first gasket 550 facing the fixing gasket 140 and a fastening hole 141 into which the coupling protrusion 551 is inserted is further formed in the fixing gasket 140. In addition, (b) of FIG. 10 illustrates an example in which the fixing gasket 140 and the first gasket 550 form a single body together.

The present invention may effectively block the space between the door and the doorframe to prevent outside air from entering the indoor space when the door is closed, and may improve thermal insulation performance, thereby reducing cooling and heating costs.

Although preferred embodiments of the present invention have been described using specific terms, the specification and drawings are to be regarded in an illustrative rather than a restrictive sense in order to help understand the present disclosure, it is obvious to those skilled in the art that various modifications and changes can be made thereto without departing from the broader spirit and scope of the disclosure and such modifications and changes should not be understood individually from the technical spirit or prospect of the disclosure.

### REFERENCE NUMERALS

| | | | |
|---|---|---|---|
| 100: | DOORFRAME | 200: | DOOR |
| 300: | HINGE COVER | 400: | SEALING COVER |
| 500: | OUTSIDE AIR BLOCKING PORTION | | |

## Claims

1. A thermally insulated door assembly comprising:
a doorframe (100) installed in an opening and comprising hinge shafts 110 installed respectively on upper and lower sides thereof;
a door (200) configured to open and close the opening;
hinge covers (300) formed respectively on upper and lower sides of the door (200) and having bearings (310) into each of which the hinge shaft (110) is inserted; and
a sealing cover (400) formed on a side surface of the door (200) adjacent to the hinge shafts (110).

2. The thermally insulated door assembly of claim 1, wherein each of the hinge covers (300) comprises a first metallic hinge cover frame (341), a second metallic hinge cover frame (342), and a hinge cover insulation portion (340) configured to connect the first and second hinge cover frames 341 and 342.

3. The thermally insulated door assembly of claim 1, wherein
the door (200) comprises a door frame (220) which surrounds the door body 210, is fixed to the door body (210), and has a first insulation portion (221) formed therein and
the first insulation portion (221) comprises a first insulation plate (221-1) configured to partition an inner space of the first insulation portion (221); and a second insulation plate (221-2) spaced apart by a predetermined distance from the first insulation plate (221-1).

4. The thermally insulated door assembly of claim 2, wherein the door (200) further comprises a frame cover (230) spaced apart by a predetermined distance from the door frame (220); and a pair of finishing plates (240) fixed to fixing protrusions (220-1) and (230-1) respectively formed on the door frame (220) and the frame cover (230),
the second insulation portion (231) disposed outside the door frame 220 is further comprised, and
the second insulation portion (231) comprises
an inner frame (231-1) provided between the outside of the door frame (220) and the frame cover (230);
side insulation portions (231-2) formed on both sides of the inner frame (231-1) to seal a space between the door frame (220) and the frame cover (230) and having an air layer a formed therein; and
close-contact insulation portions (231-3), respectively, in close contact with a front surface and a rear surface of the inner frame (231-1) to reduce conductivity of heat to the frame cover (230) and the door frame (220).

5. The thermally insulated door assembly of claim 1, wherein the sealing cover (400) comprises a first cover portion (140) configured to surround a door body (210) and having one side fixed to a door frame (220) fixed to the door body (210); and a second cover portion (420) spaced apart by a predetermined distance from the door frame (220) and having one side fixed to the door frame (220) or a frame cover (230).

6. The thermally insulated door assembly of claim 5, wherein the sealing cover (400) further comprises a connection cover portion (430) configured to connect the first and second cover portions.

7. The thermally insulated door assembly of claim 1, further comprising an outside air blocking portion (500) installed between the doorframe (100) and the sealing cover (400) to block outside air,
wherein the outside air blocking portion (500) comprises a fixed body (510) fixed to a side surface of the doorframe (100); and a rotating body (520) fixed to the first cover portion (410) and configured to come in contact with or apart from the fixed body (510) according to the opening and closing of the door (200) and
a plurality of recessed portions and groove portions are formed on each of contact surfaces of the fixed body (510) and the rotating body (520).

8. The thermally insulated door assembly of claim 7, wherein
the second cover portion (420) is rotatably coupled to the frame cover (230) and
the thermally insulated door assembly further comprises an elastic support portion (440) configured to elastically support the second cover portion (420) between the first cover portion (410) and the door frame (220).

9. The thermally insulated door assembly of claim 8, wherein the elastic support portion (440) comprises a first elastic body (441) and a second elastic body (442) to support one side of the first cover portion (410) uniformly from both sides.

10. A thermally insulated door assembly comprising:
a doorframe (100) installed in an opening;
a door (200) configured to open and close the opening;
a door frame (220) disposed on an outer side B of the door (200) and firmly fixed to a door body (210);
a pair of fixing bars (223) symmetrical to each other and installed inside the door frame (220);
a first insulation portion (221) disposed between the pair of fixing bars (223) and inserted into and fixed to an end of each fixing bar (223) of the pair; and
a plate-shaped frame cover (230) comprising a third insulation portion (234) on an indoor side of the door (200) and spaced apart by a predetermined distance from the door frame (220).

11. The thermally insulated door assembly of claim 10, further comprising:
a first side insulation portion (235) and a second side insulation portion (236) that are disposed on both sides of the frame cover (230) and configured to seal a space between the frame cover (230) and the door frame (220);
a pair of fixing protrusions (220-1) and (230-1) respectively formed on the door frame (220) and the frame cover (230) such that one fixing protrusion of the pair is disposed on an outer side of the door (200) and the other fixing protrusion is on an indoor side of the door (200);
a first gasket (550) on an inner side surface of the doorframe (100); and
a second gasket (560) on a side surface of the door (200).

12. The thermally insulated door assembly of claim 11, wherein the second side insulation portion (236) and the second gasket (560) that are adjacent to the doorframe (100) are configured to form a single body together.

13. A thermally insulated door assembly comprising:
a doorframe (100) installed in an opening and comprising hinge shafts (110) installed respectively on upper and lower sides thereof;
a door (200) configured to open and close the opening; and
a sealing cover (400) formed on a side surface of the door (200) adjacent to the hinge shafts (110),
wherein the sealing cover (400) comprises
a first cover portion (140) configured to surround a door body (210) and having one side fixed to a door frame (220) fixed to the door body (210); and
a second cover portion (420) spaced apart by a predetermined distance from the door frame (220) and having one side fixed to the door frame (220) or a frame cover 230.

14. A thermally insulated door assembly comprising:
a doorframe (100) installed in an opening and comprising hinge shafts (110) installed respectively on upper and lower sides thereof;
a door (200) configured to open and close the opening; and
hinge covers (300) formed respectively on upper and lower sides of the door (200) and having bearings (310) into each of which the hinge shaft (110) is inserted,
wherein each of the hinge covers (300) comprises a first metallic hinge cover frame (341), a second metallic hinge cover frame (342), and a hinge cover insulation portion (340) configured to connect the first and second hinge cover frames (341) and (342).

15. A thermally insulated door assembly comprising:
a doorframe (100) installed in an opening; and
a door (200) configured to open and close the opening,
wherein the door (200) comprises a door frame (220) surrounding a door body (210), fixed to the door body (210), and having a first insulation portion (221) formed therein and
the first insulation portion (221) comprises a first insulation plate (221-1) configured to partition an inner space of the first insulation portion (221); and a second insulation plate (221-2) spaced apart by a predetermined distance from the first insulation plate (221-1).
